# EUROPEAN PATENT APPLICATION

(11) **EP 4 595 892 A1**
(43) Date of publication of application: **06.08.2025**
(21) Application number: 25155879.7
(22) Date of filing: 04.02.2025
(51) Int. Cl.: A61B 6/04, A61B 6/00, A61B 6/51

(54) **X-RAY IMAGING APPARATUS**

(30) Priority: 05.02.2024 KR 20240017397
(71) Applicant: Vatech Co., Ltd., Hwaseong-si, Gyeonggi-do 18449 (KR); Vatech Ewoo Holdings Co., Ltd., Hwaseong-si, Gyeonggi-do 18449 (KR)
(72) Inventor: CHOI, Sung Il, 18449 Hwaseong-si (KR); JANG, Won Park, 18449 Hwaseong-si (KR); HWANG, Jun Sung, 18449 Hwaseong-si (KR); HWANG, Seon, 18449 Hwaseong-si (KR)
(74) Representative: Lorenz & Kollegen

(57) **Abstract**

Proposed is an X-ray imaging apparatus including a column extending in a vertical direction, a support arm extending to a side of the column, a rotary arm rotatably connected to the support arm, an X-ray generator and an X-ray detector provided at opposite ends of the rotary arm and facing each other, and a subject support part configured to extend to the side of the column and align a subject to an imaging position between the X-ray generator and the X-ray detector, wherein the subject support part is switched and attached/detached to have first and second connection states with respect to the column, and in the first and second connection states, the subject is aligned to the imaging position with an examinee placed in different positions.

## Description

### CROSS REFERENCE TO RELATED APPLICATION

The present application claims priority to Korean Patent Application No. 10-2024-0017397, filed February 05, 2024, the entire contents of which is incorporated herein for all purposes by this reference.

### BACKGROUND

### Technical Field

The present disclosure relates to an X-ray imaging apparatus and, more particularly, to an X-ray imaging apparatus that can acquire X-ray images of a subject even if an examinee is placed in different positions relative to the apparatus without moving an imaging part including an X-ray generator and an X-ray detector due to switching and attaching/detaching of a subject support part.

### Description of the Related Art

X-ray imaging is a radiographic method that utilizes the penetration ability and straightness of X-rays. In X-ray imaging, the difference in X-ray transmittance depending on the internal structures of an object is displayed as a gray level X-ray image based on the X-ray attenuation accumulated during the process of penetrating the object.

X-ray images can be classified in various ways. For example, in the field of dentistry, where teeth and tissues surrounding teeth are the area of interest, X-ray panorama images and computed tomography (CT) images of the oral and maxillofacial are mainly used.

As the types of X-ray images become more diverse, so-called multimodality X-ray imaging devices, which can acquire different X-ray images with a single device, have been introduced and are widely used. For example, in dentistry, an X-ray panoramic and CT combined X-ray imaging device is used, which can acquire X-ray panoramic images and CT images with a single device.

When imaging with a typical dental X-ray imaging device, a subject, which is the head of an examinee, is supported on a subject support part called a handle frame and aligned at the imaging position, and the opposing X-ray generator and X-ray detector rotate with the subject in between to acquire an X-ray panorama and/or CT image.

A typical dental X-ray imaging device may consist of column extending up and down, an arm extending to one side from the column, a subject support part connected to the arm, a support arm extending from one side of the top of the column, and an imaging part connected to the support arm. The imaging part has an X-ray generator and an X-ray detector arranged to face each other on opposite sides of a rotating arm rotatably connected to the support arm. The subject support part serves to align a subject at the imaging position, which is a point between the X-ray generator and the X-ray detector.

Thus, when taking an X-ray image, the examinee places the subject (head) on the subject support part with the column on his or her left or right side, so that the subject is located in the imaging area between the X-ray generator and the X-ray detector, and the X-ray generator and the X-ray detector rotate and/or move with the subject in between to acquire X-ray images.

Meanwhile, in a dental clinic, an imaging device like the above is disposed in a shielded room, but because it is difficult to allocate a lot of space to the shielding room and the shape of the space is different, entry and movement path of an examinee becomes complicated depending on the structure of an X-ray imaging device, especially depending on the position of the examinee relative to the X-ray imaging device when taking X-ray images.

Accordingly, an X-ray imaging system that can image a subject using a single imaging device even if an examinee's position changes with respect to the device is required.

The technology behind this application is disclosed in Korean Patent Application Publication No. 10-2020-0007303.

### SUMMARY

Accordingly, the present disclosure has been made keeping in mind the above problems occurring in the related art, and the present disclosure is intended to provide an X-ray imaging apparatus that acquires X-ray images of a subject even if an examinee is placed in different positions relative to the apparatus without moving an imaging part including an X-ray generator and an X-ray detector due to switching and attaching/detaching of a subject support part.

In addition, an objective of the present disclosure is to provide an X-ray imaging apparatus that allows sufficient space to be secured for an examinee entering a shielded room for imaging despite the structure of the shielded room, etc., due to switching and attaching/detaching of the subject support part.

However, the objectives sought to be achieved by the embodiments of the present disclosure are not limited to the ones described above, and other objectives may exist.

In order to achieve the above objectives, according to an embodiment of the present disclosure, there is provided an X-ray imaging apparatus including: a column extending in a vertical direction; a support arm extending to a side of the column; a rotary arm rotatably connected to the support arm; an X-ray generator and an X-ray detector provided at opposite ends of the rotary arm and facing each other; and a subject support part configured to extend to the side of the column and align a subject to an imaging position between the X-ray generator and the X-ray detector, wherein the subject support part may be switched and attached/detached to have first and second connection states with respect to the column, and in the first and second connection states, the subject may be aligned to the imaging position with an examinee placed in different positions.

In addition, according to an embodiment of the present disclosure, the subject support part may align the subject at the imaging position from a first side of a first direction of the column in the first connection state, and may align the subject at the imaging position from a second side of the first direction of the column in the second connection state.

In addition, according to an embodiment of the present disclosure, the subject support part may include: an arm connected to the first side of the column; and a support frame that is connected to an arm and supports the subject to align the subject to the imaging position.

In addition, according to an embodiment of the present disclosure, at least one of the arm and the support frame may be switched up and down or left and right in each of the first and second connected states.

In addition, according to an embodiment of the present disclosure, the arm may be connected to the column by switching up and down in each of the first and second connection states.

In addition, according to an embodiment of the present disclosure, the support frame may be connected to the column by switching left and right in each of the first and second connected states.

In addition, according to an embodiment of the present disclosure, the apparatus may further include a guide part provided on an upper surface of the support frame to guide the subject to the imaging position.

In addition, according to an embodiment of the present disclosure, the guide part may include at least one of: a support end that supports a lower end of the subject and aligns the subject to the imaging position; and supports that respectively adhere closely to both left and right ends of the subject and align the subject to the imaging position.

The above-described technical solutions are merely illustrative and should not be construed as intended to limit the present disclosure. In addition to the above-described exemplary embodiments, additional embodiments may be present in the drawings and detailed description of the present disclosure.

According to the above-mentioned technical solutions of the present disclosure, provided is an X-ray imaging apparatus that can acquire X-ray images of a subject even if an examinee is placed in different positions relative to the apparatus without moving an imaging part including an X-ray generator and an X-ray detector due to switching and attaching/detaching of a subject support part.

Furthermore, provided is an X-ray imaging apparatus that can allow sufficient space to be secured for an examinee entering a shielded room for imaging despite the structure of the shielded room, etc., due to switching and attaching/detaching of the subject support part.

However, the effects achieved by the embodiments of the present disclosure are not limited to the ones described above, and other effects may exist.

### BRIEF DESCRIPTION OF THE DRAWINGS

The above and other objectives, features, and other advantages of the present disclosure will be more clearly understood from the following detailed description when taken in conjunction with the accompanying drawings, in which:
FIGS. 1 and 2 are schematic conceptual views of an X-ray imaging apparatus according to an embodiment of the present disclosure;
FIG. 3 is a view schematically showing how an X-ray imaging apparatus according to an embodiment of the present disclosure acquires an X-ray image of a subject;
FIG. 4 is a schematic perspective view of a subject support part according to an embodiment of the present disclosure;
FIG. 5 is a schematic exploded perspective view of the subject support part in a first connected state;
FIG. 6 is a schematic exploded perspective view of the subject support part in a second connected state;
FIG. 7 is a view schematically showing the connection between a support and an operation dial according to an embodiment of the present disclosure; and
FIG. 8 is a view schematically showing a base according to an embodiment of the present disclosure.

### DETAILED DESCRIPTION

Hereinafter, embodiments of the present disclosure will be described in detail with reference to the drawings so that those skilled in the art can easily implement the embodiments. However, the present disclosure may be implemented in various different forms and is not limited to the embodiments described herein. In addition, in order to clearly explain the present disclosure in the drawings, parts not related to the description have been omitted, and similar parts have been given similar reference numerals throughout the specification.

Throughout the specification of the present application, when a part is said to be "connected" to another part, this includes not only the case where the parts are "directly connected" but also the case where the parts are "indirectly connected" with another component in between or the parts are "electrically connected" with an element in between.

Throughout the specification, when a member is said to be located "on", "above", "at the top", "beneath", "below", or "at the bottom" of another member, this includes not only the case where a member is in contact with another member, but also the case where another member exists between the two members.

Throughout the specification, when it is said that a part "includes" a certain component, this means that it may further include other components rather than excluding other components, unless specifically stated to the contrary.

Terms such as "first-" and "second-" may be used to indicate the same or substantially the same component in a different order, and may be interpreted as a component that is substantially the same as a component that does not indicate "first", "second", etc.

In addition, in the description of the embodiments of the present disclosure, terms related to direction or position (above, top, below, etc.) are set based on the arrangement status of each component shown in the drawings.

Hereinafter, an X-ray imaging apparatus 1000 according to an embodiment of the present disclosure will be described. The X-ray imaging apparatus 1000 according to an embodiment of the present disclosure is an X-ray imaging apparatus that can align a subject, which is the head of an examinee, to the imaging position even if the examinee is placed in different positions due to switching and attaching/detaching of a subject support part. The apparatus may acquire X-ray images without moving an imaging part with the examinee placed in different positions.

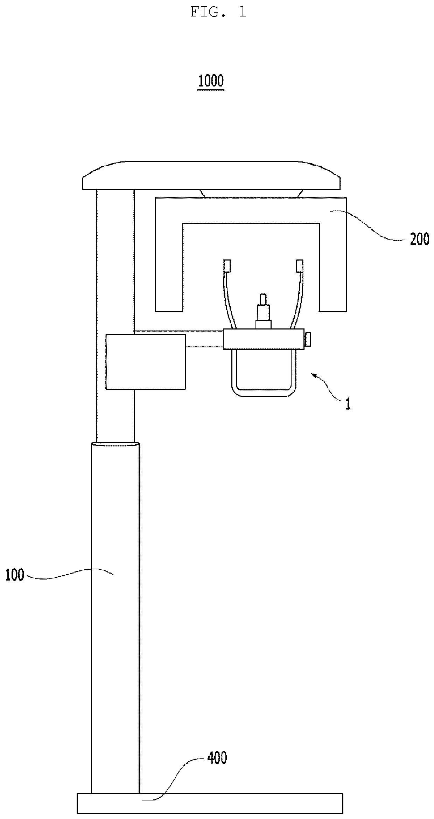

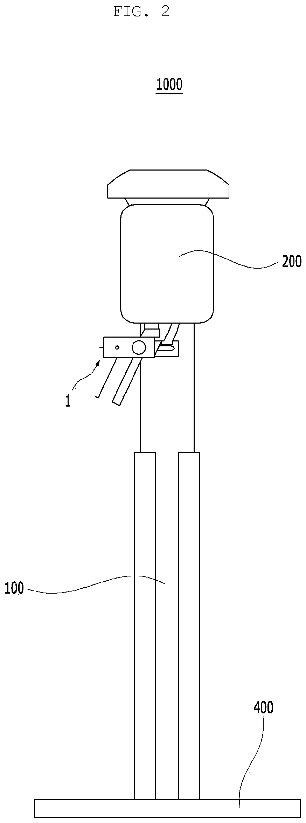

First, referring to FIGS. 1 and 2, the X-ray imaging apparatus 1000 according to an embodiment of the present disclosure may include: a column 100 extending vertically; a support arm 240 connected to the top of the column 100; an imaging part 200 connected to the support arm 240 to acquire an X-ray image; a base 400 provided at the bottom of the column and installed in contact with the floor surface to support the apparatus; and a subject support part 1 to be described later. The X-ray imaging apparatus 1000 may further include a lifting member that is movable in the vertical direction with respect to the column and moves the subject support part 1 and the imaging part 200 in the vertical direction.

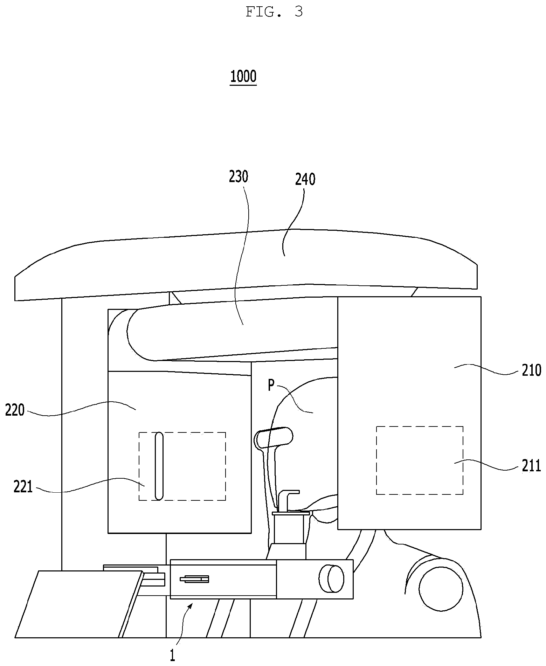

Referring to FIG. 3, the imaging part 200 may consist of: a first extension part 210 where an X-ray generator 211 is installed; a second extension part 220 where an X-ray detector 221 is installed and is disposed to face the first extension part 210; and a rotary arm 230 connecting the first extension part 210 and the second extension part 220 in the horizontal direction, and rotatably connected to the support arm 240.

The imaging part 200 of the above structure is configured such that, when a subject is aligned at the imaging position between the first and second extension parts 210 and 220, as the rotary arm 230 rotates around the rotation axis, the X-ray generator 211 and the X-ray detector 221 rotate with a subject P in between to acquire an X-ray image.

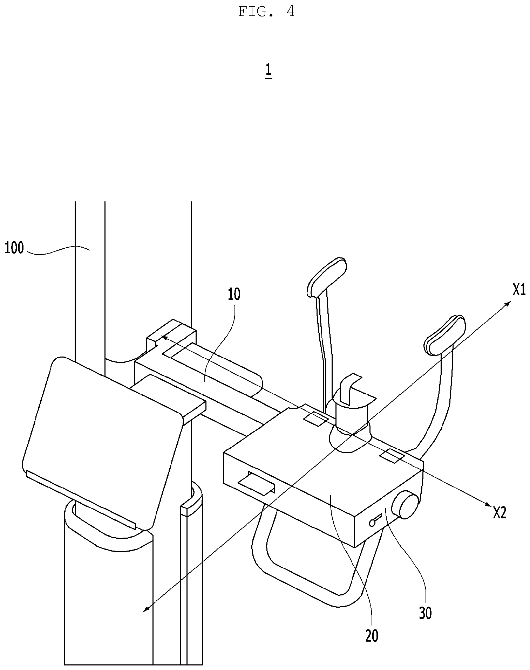

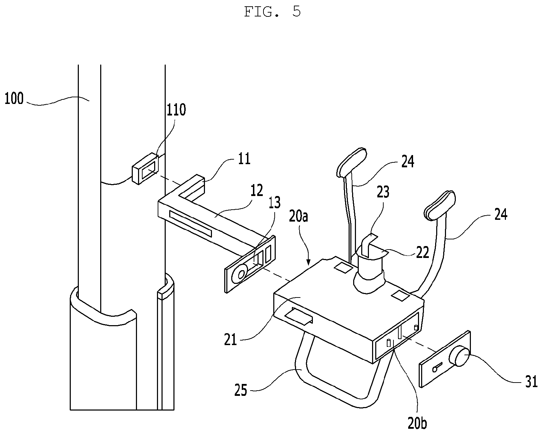

Referring to FIGS. 4 and 5, the subject support part 1 according to an embodiment of the present disclosure may include: an arm 10 that is detachably connected to the column 100 beneath the support arm 240 and extends to one side of the column 100; a support frame 20 detachably connected to one end of the arm 10; and a cover 30 detachably coupled to one side of the support frame 20.

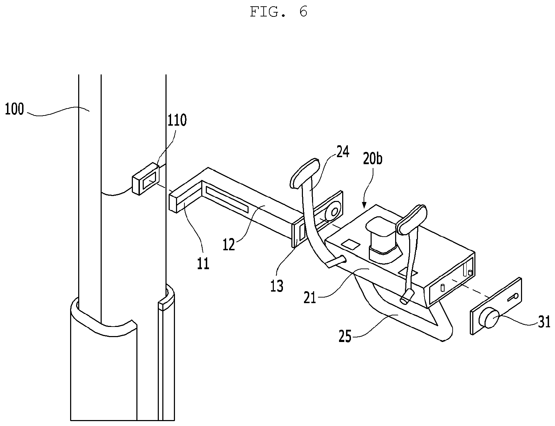

In addition, referring to FIGS. 4 and 6, the subject support part 1 of the present disclosure may have: a first connection state in which the subject P is guided to the imaging position with an examinee located on one side of a first direction (X1 direction); and a second connection state in which the subject P is guided to the imaging position with an examinee located on the other side of a first direction (X1 direction). That is, as described later, the arm 10 and the support frame 20 may be provided to be switchable between the first connection state that guides the alignment of the subject on one side in the first direction and the second connection state that guides the alignment of the subject on the other side in the first direction. That is, the subject support part 1 may be switched and attached/detached to have first and second connection states with respect to the column, and in each of the first and second connection states, the examinee may align the subject to the imaging position from different positions. In an embodiment, the subject support part 1 may align the subject to the imaging position with the examinee placing the column on one side of him or her in the first connection state, and may align the subject to the imaging position with the examinee placing the column on the other side of him or her in the second connection state.

The arm 10 is connected to one side of the column and may extend from the column 100 in a second direction (X2 direction). The arm may support the support frame 20 by connecting the column 100 and the support frame 20 between the column 100 and the support frame 20. The arm 10 may include a connection part 11, an extension rod 12, and a detachable part 13.

The connection part 11 mediates the connection between the arm 10 and the column 100, and the arm 10 may be coupled to a connection member 110 of the column 100 through the connection part 11. The arm 10 may be provided to be detachable from the connection member 110, or may be provided to be rotatable about the connection member 110. After the arm 10 is disconnected from the connection member 110, the arm 10 may be rotated 180 degrees, that is, upside down, about an axis extending in the second direction, and then be coupled to the connection member 110 again. Alternatively, the arm 10 may rotate 180 degrees about an axis extending in the second direction while coupled to the connection member 110.

The connection part 11 may be detachably coupled to the connection member 110 through various mechanical coupling methods such as fit coupling and tapered coupling. For example, the connection member 110 may be a coupling hole disposed at the center of the column 100 in the first direction.

The extension rod 12 may extend along the second direction (X2 direction) between the column 100 and the support frame 20. In an embodiment, the extension rod 12 may extend along the second direction from one end of the connection part 11 in the first direction as shown in FIGS. 4 to 6.

The detachable part 13 may be detachably connected to the side of the support frame. In the first connection state, the detachable part 13 may be detachably connected to a first side 20a of the support frame in the second direction, whereas in the second connection state, the detachable part 13 may be detachably connected to a second side 20b in the second direction, which is opposite to the first side 20a in the second direction of the support frame. The detachable part 13 and both sides 20a and 20b of the support frame in the second direction may adopt a predetermined mechanical coupling method. As in the case of the connection part 11, the coupling between the detachable part 13 and either side 20a or 20b of the support frame may be understood as being detachable.

Meanwhile, an area imaged by the imaging part 200 is offset by a predetermined distance from the center of the support frame 20, rather than the center of the support frame 20, toward the first direction.

When the center of the support frame 20 does not move along the first direction in a situation where the subject support part 1 is switched between the first connection state and the second connection state, the subject P is guided to different positions relative to the imaging part in the first connection state and the second connection state. Thus, by allowing the detachable part 13 to be offset a predetermined distance along the first direction with respect to the connection part 11 or the connection member 110, and allowing the center of the support frame 20 to move a predetermined distance along the first direction when the subject support part 1 is switched between the first connection state and the second connection state, an X-ray image of the subject P may be acquired in the same imaging area without moving the imaging part 200 even if the position of the examinee changes.

In the same manner, in the first connection state (see FIG. 5) and in the second connection state (see FIG. 6), the arm 10 may be formed to be axisymmetric about an axis X2 extending in the second direction from the connection part 11. To this end, the portion where the detachable part 13 is coupled to the side of the support frame may be formed to be vertically symmetrical.

The support frame 20 is detachably connected to one end of the arm 10 to support the subject and align the subject to the imaging position.

The support frame 20 may guide the alignment of the subject P on one side of the first direction. That is, the support frame 20 may position the subject P on one side in the first direction in the first connection state, and may position the subject P on the other side in the first direction in the second connection state.

To this end, the support frame 20 may be provided with both sides 20a and 20b in the second direction respectively connectable to the arm. That is, the support frame 20 may be switched left and right so that in the first connection state, the first side 20a is connected to the detachable part 13, and in the second connection state, the second side 20b is connected to the detachable part 13.

It should be understood that at least one of the arm 10 and the support frame 20 is switched up and down or left and right in each of the first and second connected states. To be specific, the arm 10 may be connected to the column by up-and-down switching in each of the first and second connection states, and the support frame 20 may be connected to the arm 10 by left-and-right switching in each of the first and second connection states.

In addition, the support frame 20 may be symmetrical left and right based on an axis X1 that passes through the center thereof and extends along the first direction. Accordingly, the support frame 20 is able to align the position of the subject P on opposite sides in the first direction no matter which of the two sides 20a and 20b in the second direction is connected to the detachable part 13.

Referring to FIGS. 5 and 6, the subject support part 1 may include a guide part that is provided on the support frame 20 and guides the subject to the imaging position. In an embodiment, the guide part may include: a support end 22 that is seated on the support frame 20 and supports the chin, which is the lower part of the subject P; and a bite 23 formed to extend from the support end 22 so as to be bitten by the examinee with the mouth. In addition, in an embodiment, the guide part may include a pair of supports 24, each support 24 being placed on each of the left and right sides of the subject, with the distance between the supports 24 decreasing so that the supports 24 may come in close contact with the temples, etc., or increasing. The guide part may also include a handle 25 provided on the lower side of the support frame 20 so as to be held by the examinee.

The support frame 20 may be equipped with a built-in connection shaft 24a to adjust the spacing between the pair of supports 24. For example, the connection shaft 24a may be a two-way ball screw with threads in opposite directions formed on the left and right sides with respect to the center thereof, and the supports 24 are screws respectively fastened to the left and right sides of the connection shaft 24a. Thus, when the connection shaft 24a rotates in one direction, the gap between the supports 24 becomes wider, and when the connection shaft 24a rotates in the opposite direction, the gap between the supports 24 becomes narrower.

Continuing to refer to FIGS. 4 to 6, the cover 30 may be detachably connected to the side of the support frame 20 in the direction opposite to the arm 10. That is, in the first connection state, the detachable part 13 of the arm may be detachably connected to the first side 20a of the support frame in the second direction, and the cover 30 may be detachably connected to the second side 20b in the second direction of the support frame. In the second connection state, the detachable part 13 of the arm may be detachably connected to the second side 20b of the support frame in the second direction, and the cover 30 may be detachably connected to the first side 20a in the second direction of the support frame.

In an embodiment, a control dial 31 connected to the connection shaft 24a may be provided on the cover 30. For example, the control dial 31 may be a rotary dial formed on one side of the cover 30. Thus, depending on the direction in which the control dial 31 is turned, the distance between the supports 24 may become farther or closer.

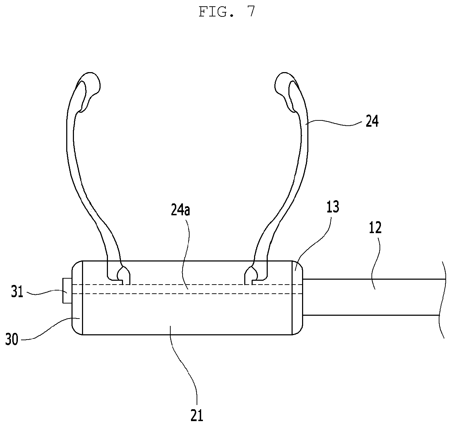

Referring to FIG. 7, in the first or second connection state, the cover 30 and the control dial 31 may be coupled to the first side 20a or the second side 20b of the support frame in the second direction, and the connection shaft 24a may extend from the supports 24 to opposite sides in the second direction. As a result, the control dial 31 may be connected to the connection shaft 24a on the first side 20a or the second side 20b of the support frame in the second direction.

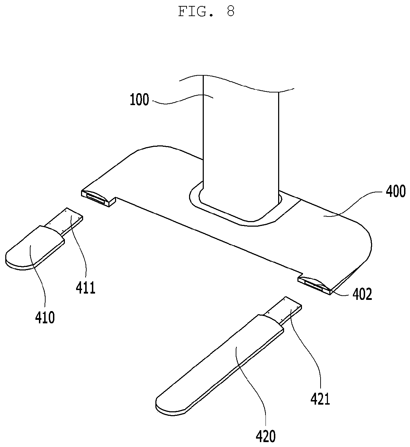

Referring to FIG. 8, the base 400 is provided at the bottom of the column 100 and may stably support the X-ray imaging apparatus 1000 of the present disclosure to prevent the apparatus 1000 from falling over due to external force.

In an embodiment, the base 400 may include a first foot 410 and a second foot 420 extending along the direction in which the arm 10 extends.

The first foot 410 and the second foot 420 may be spaced apart from each other with the examinee in between, and may be detachably connected to the base 400. As shown in FIG. 8, the first foot 410 and the second foot 420 may include base coupling parts 411 and 421 respectively coupled to coupling holes 401 and 402 formed on one side of the base. For easy attachment to and detachment from the base 400, the base coupling parts 411 and 421 and the coupling holes 401 and 402 may be attached and detached by a method such as fit coupling.

In addition, the first foot 410 and the second foot 420 may have different lengths to allow the examinee to easily enter the imaging zone, and the first foot, which is short in length, may be arranged to be located on the side of the path through which the examinee enters. That is, the first foot 410 and the second foot 420 may be coupled to the base 400 depending on the guide direction for examinee entry according to the first connection state and the second connection state. It should be understood that when guiding the examinee's entry to one side of the first direction, the first foot, which is relatively short in length, is placed on one side of the first direction.

The above-described description of the present disclosure is for illustrative purposes, and those skilled in the art will understand that the present disclosure can be easily modified into other specific forms without changing the technical idea or essential features thereof. Therefore, the embodiments described above should be understood in all respects as illustrative and not restrictive. For example, each component described as single may be implemented in a distributed manner, and similarly, components described as distributed may also be implemented in a combined form.

The scope of the present disclosure is indicated by the appended claims rather than the detailed description above, and the meaning and scope of the patent claims and all changes or modified forms derived from the equivalent concept thereof should be construed as being included in the scope of the present disclosure.

## Claims

1. An X-ray imaging apparatus comprising:
a column extending in a vertical direction;
a support arm extending to a side of the column;
a rotary arm rotatably connected to the support arm;
an X-ray generator and an X-ray detector provided at opposite ends of the rotary arm and facing each other; and
a subject support part configured to extend to the side of the column and align a subject, which is part of an examinee, to an imaging position between the X-ray generator and the X-ray detector,
wherein the subject support part is switched and attached/detached to have first and second connection states with respect to the column, and in the first and second connection states, the subject is aligned to the imaging position with the examinee placed in different positions.

2. The apparatus of claim 1, wherein in the first connection state, the examinee is positioned at one side of the column to align the subject to the imaging position, and in the second connection state, the examinee is positioned at the other side of the column to align the subject to the imaging position.

3. The apparatus of claim 1, wherein the subject support part comprises:
an arm connected to the first side of the column; and
a support frame that is connected to an arm and supports the subject to align the subject to the imaging position.

4. The apparatus of claim 3, wherein at least one of the arm and the support frame is switched up and down or left and right in each of the first and second connected states.

5. The apparatus of claim 3, wherein the arm is connected to the column by switching up and down in each of the first and second connection states.

6. The apparatus of claim 3, wherein the support frame is connected to the column by switching left and right in each of the first and second connected states.

7. The apparatus of claim 3, further comprising:
a guide part provided on an upper surface of the support frame to guide the subject to the imaging position.

8. The apparatus of claim 7, wherein the guide part comprises at least one of:
a support end that supports a lower end of the subject and aligns the subject to the imaging position; and
supports that respectively adhere closely to both left and right ends of the subject and align the subject to the imaging position.
